# EUROPEAN PATENT SPECIFICATION

(11) **EP 0 666 081 B1**
(45) Date of publication and mention of the grant of the patent: **20.10.2004**
(21) Application number: 95200138.6
(22) Date of filing: 20.01.1995
(51) Int. Cl.: A61L 15/28, A61L 15/60

(54) **Wound dressing**
Wundverband
Pansement

(30) Priority: 24.01.1994 GB 9401279
(43) Date of publication of application: 09.08.1995
(73) Proprietor: Bristol-Myers Squibb Company, New York, N.Y. 10154 (US)
(72) Inventor: Court, Andrew David, Wirral, Merseyside L64 0UL (GB); Hislop, Allan Brian, Lache Park, Chester CH4 8HW (GB); Queen, Douglas, Wirral, Merseyside L61 9PT (GB)
(74) Representative: Mays, Julie

(56) References cited:
- EP-A- 0 208 395
- EP-A- 0 532 275
- EP-A- 0 567 311
- WO-A-90/14110
- US-A- 3 249 109
- US-A- 4 292 972

## Description

This invention relates to a wound dressing. More particularly, this invention relates to a wound dressing comprising a fibrous material coated with an impregnating composition, useful for treating wounds.

The invention also relates to a method of treating a wound comprising applying the dressing to a wound.

It is well known that the cleansing and debriding of wounds and the removal of wound exudate is important to the process of healing wounds. Commonly used wound dressings comprise gauze, foams, sponges, cotton wads or other fibrous materials. Gauze and other fibrous materials absorb fluids by capillary action with the disadvantage that when new tissue is formed as part of the healing process, it engulfs the fibres and is torn when the material is removed causing wound injury. Many fibrous products also have the disadvantage that they do not conform readily to the shape of the wound particularly if the wound includes a cavity.

Saline coated gauzes are known in the art but have the disadvantage that the coatings are mobile and can be lost from the gauze and wound. In addition many of the coated gauzes cannot be used on exudating wounds as the coatings have limited absorbency.

WO 90/14110 discloses a pharmaceutical preparation which comprises a stable suspension of a water insoluble alginate for external application to a patient. A colloidal suspension of calcium alginate is conveniently stabilized by use of xantham gum, and/or gellan gum, and/or locust bean gum which act as suspending and gelling agents. The preparation may have a variety of physical forms, ranging from a thin runny gel capable of direct application to the body surface, through a thick gel, to a self-supporting slab, pad or wafer of desired size, shape and thickness which may be held in place with a surgical dressing. The preparation may also be presented in the form of a surgical dressing which carries, e.g. is impregnated with or contains in slab or wafer from, a preparation of the invention. The alginate is haemostatic and also has healing and regranulation properties. The preparation can thus be used in treatment of bleeding wounds and injuries and also non-bleeding such as burns, ulcers and many other conditions.

It is also known from EP 0 567 311 A2 (Queen/ER Squibb and Sons) to use an absorbent gel to remove exudate from wounds. The gel is packaged in a tube and is applied to the wound from the tube. For some wounds this method of application has the disadvantages of needing a secondary dressing and being time consuming and messy. We have now found that a dressing comprising a fibrous material coated with a composition applied as a particular gel, mitigates many of the above disadvantages.

Accordingly the invention comprises a wound dressing comprising a fibrous material impregnated with a compositon characterised in that the composition comprises:
(a) from about 0.05% to 10% by weight of a natural gelling agent;
(b) from about 1.0% to 10% by weight of a hydrocolloid;
(c) from about 5.0% to 30.0% by weight of a glycol and
(d) at least 50% by weight of water.

The most preferred composition contains 0.1% pectin, 3.4% sodium carboxymethyl cellulose, 15% propylene glycol and 81.5% water.

The natural gelling agent is preferably selected from pectin, alginic acid and salts thereof, carageenan, tragacanth, acacia, locust bean gum, guar gum, starch, agar and gelatin. More preferably the pectin is pectin with a high ester content derived from citrus peel consisting chiefly of the partial methyl esters of polygalachronic acid (approximately 65% of the carboxyl groups are esterified). Representatives of the pectin useful in the gel composition is that marketed under the name GENU pectin type VIS-L by Copenhagen Pectin. The natural gelling agent is preferably present in an amount from 0.05% to 1.0% by weight.

The hydrocolloid is preferably selected from sodium carboxymethyl cellulose, methyl cellulose, hydroxypropylmethyl cellulose, hydroxyethyl cellulose, hydroxymethylcellulose, hydroxypropyl cellulose, carboxyvinyl polymer and salts thereof, poloxamer for example Pluronic F127, xanthan gum, povidone, modified starches, and guar derivatives. The carboxymethyl cellulose is preferably sodium carboxymethyl cellulose present in an amount from about 2.0% to 4.5% by weight. The preferred sodium carboxymethyl cellulose is a high viscosity sodium carboxymethyl cellulose (typically in the range 2000 - 4500 cps as measured by Brookfield LV Viscometry of a 1% solution, oven dry basis, 25°C and spindle 4/30 rpm.

The glycol can be an aryl or alkylene glycol, preferably selected form the group of glycerol, polyethylene glycol, panthanol, and sorbitol. If the glycol is an alkylene glycol it is preferably propylene glycol present at from about 10.0% to 20.0% by weight.

The water used in the present invention is preferably purified and pyrogen free water and is present in an amount sufficient to bring the total composition up to 100% by weight.

Various optional ingredients can also be included in the final composition such as preservatives eg, methylhydroxybenzoate and propylhydroxybenzoate. In addition, the wound gel composition can, if desired, contain small amounts (effective amounts) i.e. less than 5%, of pharmacologically active ingredients. For example, an antibiotic or antimicrobial agent such as metronidazole, silver sulphadiazine, neomycin or penicillin, and antiseptic agent such as povidone iodine, and antiinflammatory agent such as hydrocortisone or triamcinolone acetonide, or a skin protective agent such a zinc oxide can be included.

The compositon used to make the dressing of this invention is prepared by mixing the natural gelling agent in water with heating (for example at a temperature from about 50°C to 60°C) to form a solution. Alkylene glycol is added while mixing and hydrocolloid gradually added while mixing to form a gel.

We have found that the wound dressing of the invention performs particularly well if the gel has a viscosity of from 50 to 800 Pas as determined by Viscolog model MRV8 viscometer and a helical drive unit and PD spindal rotating at 2.5 rpm. If this is achieved flow of the gel off the gauze either in the packaging or at the wound site is reduced while conformity of the dressing to the surface of the wound is optimised. Since the gel has some wet tack the gauze stays in place more readily.

For certain types of wound treatment it is advantageous to have a dressing comprising many layers of substrate. For such a dressing the impregnating composition needs to be more mobile than a gel to fully impregnate the substrate. This can be achieved with the present invention by sterilising a gel impregnated fibrous substrate by irradiation which then destroys the gel-structure and renders the composition more mobile. The advantage of using a gel to impregnate the substrate is that the gel gives processing advantages in that it can be extruded onto the substrate and less coating is lost from the substrate during packaging. The resultant, irradiated dressing comprises a composition which includes hydrocolloids which are able to absorb wound exudate. This is an advantage over known saline impregnated dressings which rely on the gauze to absorb exudate with the unwanted result of wound re-injury.

The fibrous material of the invention may be selected from viscose, cotton and polyester in the form of a knitted, woven or non woven sheet or ribbon, but is preferably a knitted gauze. Most preferably the gauze is a knitted viscose being a warp knitted fabric using 220 Dtex, 40 filament Bright viscose yarn. For certain applications the substrate is preferably a multiplied plain woven cotton gauze.

The wound dressing of this invention is prepared by coating the fibrous material with the impregnating compositon preferably by extruding the composition in the form of a gel through a nozzle onto both sides of the gauze. Preferably the coating density is from 750g to 2kg of gel per square metre of gauze.

The wound dressing may then be packaged in a moist state in a foil envelope and sterilized. Sterilization can be achieved either by autoclaving or by irradiating the envelope.

The invention will now be illustrated by the following nonlimiting examples.

### Example 1

A wound dressing was prepared by coating a 10cm square of gauze made from knitted viscose with the gel composition below to a density of 1kgm⁻².

| Gel composition | % by weight |
|---|---|
| Pectin | 0.1% |
| Sodium carboxymethyl cellulose | 3.4% |
| Propyleneglycol | 15.0% |
| Purified water | 81.5% |

Pectin (0.2g) was added to purified water (163.0g) in a beaker and heated to 50 - 60°C with constant stirring until the pectin dissolved. Propylene glycol (30.0g) was added and sodium carboxymethyl cellulose (6.8g) was gradually added with constant mixing. A hydrocolloid gel (200g) was produced.

The wound dressing was packaged in a foil envelope and sterilized by autoclaving at 121°C.

The handling characteristics of the wound dressing were evaluated in a full thickness wound model. The wound dressing of the invention was compared to Tricotex (a non-adherent dressing ex Smith and Nephew) alone by applying the dressings under a secondary dressing of either Tricotex or Op-Site (ex Smith and Nephew) an occlusive dressing to a total of 18 wounds. Dressings were changed on days 3, 4, 7 and 9.

Generally when applying the dressings into the wound cavity, the wound dressing of the invention demonstrated superior handling characteristics in that it could easily be moulded to the shape of the wound. The Tricotex gauze dressing on its own was difficult to apply to the wounds and did not conform at all to the shape of the wound. A subjective evaluation of the ease of removal of the dressings from the wounds demonstrated that generally the wounds treated with Tricotex gauze under Tricotex or Op-Site showed poor removal. This was because the gauze appeared to adhere to the wound surface, and in some cases caused re-injury of the tissue on removal. The wound dressing of the invention was easily removed from the wound without causing re-injury of the wound and without the use of a saline wash, whether wound fluid was present or not.

### Example 2

The rates of contraction of the wounds of Example 1 were measured as a percentage of wound area on day 0. All the wounds with dressings under Tricotex followed a normal contraction curve. However the wounds with dressings according to the invention showed superior performance under Op-Site, for example at day 11 Tricotex under Op-Site demonstrated 85% of the original wound area while the wound dressing of the invention under Op-Site demonstrated 45% of the original wound area.

### Example 3

A wound dressing was prepared by coating a 10cm square of gauze made from plain woven cotten mulitplied to 8 to 12 ply, with the gel composition below to a density of 2kgm⁻².

| Gel composition | % by weight |
|---|---|
| Pectin | 0.1% |
| Sodium carboxymethyl cellulose | 3.4% |
| Propyleneglycol | 15.0% |
| Purified water | 81.5% |

Pectin (0.2g) was added to purified water (163.0g) in a beaker and heated to 50 - 60°C with constant stirring until the pectin dissolved. Propylene glycol (30.0g) was added and sodium carboxymethyl cellulose (6.8g) was gradually added with constant mixing. A hydrocolloid gel (200g) was produced.

The wound dressing was packaged in a foil envelope and sterilized by exposure to gamma irradiation at 25KGy. The irradiation destroyed the gel structure to allow penetration of the coating into the gauze.

## Claims

1. A wound dressing comprising a fibrous material impregnated with a composition **characterised in that** said composition comprises:
a) from 0.05% to 10% by weight of a natural gelling agent;
b) from 1.0% to 10% by weight of a hydrocolloid;
c) from 5.0% to 30.0% by weight of an alkylene glycol and
d) at least 50% by weight of water.

2. A wound dressing as claimed in claim 1 **characterised in that** the composition is in the form of a gel.

3. A wound dressing as claimed in claim 1 or claim 2 **characterised in that** the natural gelling agent is pectin.

4. A wound dressing as claimed in any preceding claim **characterised in that** the hydrocolloid is sodium carboxymethyl cellulose.

5. A wound dressing as claimed in any preceding claim **characterised in that** the alkylene glycol is propylene glycol.

6. A wound dressing agent as claimed in any preceding claim **characterised in that** the gel has a viscosity from 50 to 800 Pas.

7. Use of a fibrous material impregnated with a composition comprising:
a) from 0.05% to 10% by weight of a natural gelling agent;
b) from 1.0% to 10% by weight of a hydrocolloid;
(c) from 5.0% to 30.0% by weight of an alkylene glycol and
(d) at least 50% by weight of water in the manufacture of a medicament for use in the treatment of wounds and in particular chronic wounds.

8. Use as claimed in claim 7 **characterised in that** the composition is in the form of a gel.

9. A wound dressing product comprising a sealed foil envelope containing a wound dressing comprising a fibrous material impregnated with a composition said compostion comprising:
(a) from 0.05% to 10% by weight of a natural gelling agent;
(b) from 1.0% to 10% by weight of a hydrocolloid;
(c) from 5.0% to 30.0% by weight of an alkylene glycol and
(d) at least 50% by weight of water.

10. A product as claimed in claim 9 **characterised in that** the composition is in the form of a gel.

11. A method for making a wound dressing comprising the steps of:
(i) impregnating a fibrous material with a gel said gel comprising:
(a) from 0.05% to 10% by weight of a natural gelling agent;
(b) from 1.0% to 10% by weight of a hydrocolloid;
(c) from 5.0% to 30.0% by weight of an alkylene glycol and
(d) at least 50% by weight of water;
(ii) sealing the impregnated fibrous material in an envelope and
(iii)steralizing said envelope and contents.

12. Method as claimed in claim 11 **characterised in that** the steralization is carried out by autoclaving.

13. Method as claimed in claim 11 **characterised in that** the steralization is carried out by gamma irradiation.

## Revendications

1. Pansement comprenant un matériau fibreux imprégné avec une composition, **caractérisé en ce que** ladite composition comprend :
a) de 0,05 % à 10 % en poids d'un agent gélifiant naturel ;
b) de 1,0 % à 10 % en poids d'un hydrocolloide ;
c) de 5,0 % à 30,0 % en poids d'un alkylèneglycol ; et
d) au moins 50 % en poids d'eau.

2. Pansement selon la revendication 1, **caractérisé en ce que** la composition est sous forme d'un gel.

3. Pansement selon la revendication 1 ou la revendication 2, **caractérisé en ce que** l'agent gélifiant naturel est la pectine.

4. Pansement selon l'une quelconque des revendications précédentes, **caractérisé en ce que** l'hydrocolloïde est de la carboxyméthylcellulose de sodium.

5. Pansement selon l'une quelconque des revendications précédentes, **caractérisé en ce que** l'alkylèneglycol est le propylèneglycol.

6. Pansement selon l'une quelconque des revendications précédentes, **caractérisé en ce que** le gel a une viscosité de 50 à 800 Pa.s.

7. Utilisation d'un matériau fibreux imprégné d'une composition comprenant :
a) de 0,05 % à 10 % en poids d'un agent gélifiant naturel ;
b) de 1,0 % à 10 % en poids d'un hydrocolloïde ;
c) de 5,0 % à 30,0 % en poids d'un alkylèneglycol ; et
d) au moins 50 % en poids d'eau ;
pour la fabrication d'un médicament à utiliser dans le traitement de plaies et, en particulier, des plaies chroniques.

8. Utilisation selon la revendication 7, **caractérisé en ce que** la composition est sous forme d'un gel.

9. Produit de type pansement comprenant une enveloppe en feuille fermée hermétiquement contenant un pansement comprenant un matériau fibreux imprégné avec une composition, **caractérisé en ce que** ladite composition comprend :
(a) de 0,05 % à 10 % en poids d'un agent gélifiant naturel ;
(b) de 1,0 % à 10 % en poids d'un hydrocolloide ;
(c) de 5,0 % à 30,0 % en poids d'un alkylèneglycol ; et
(d) au moins 50 % en poids d'eau.

10. Produit selon la revendication 9, **caractérisé en ce que** la composition est sous forme d'un gel.

11. Procédé de fabrication d'un pansement comprenant les étapes consistant à :
i) imprégner un matériau fibreux avec un gel, ledit gel comprenant :
(a) de 0,05 % à 10 % en poids d'un agent gélifiant naturel ;
(b) de 1,0 % à 10 % en poids d'un hydrocolloïde ;
c) de 5,0 % à 30,0 % en poids d'un alkylèneglycol ; et
d) au moins 50 % en poids d'eau ;
ii) fermer hermétiquement le matériau fibreux hermétiquement dans une enveloppe, et
iii) stériliser ladite enveloppe et son contenu.

12. Procédé selon la revendication 11, **caractérisé en ce que** la stérilisation est réalisée par un passage en autoclave.

13. Procédé selon la revendication 11, **caractérisé en ce que** la stérilisation est réalisée par un rayonnement gamma.

## Patentansprüche

1. Wundverband, umfassend ein faserförmiges Material, das mit einer Zusammensetzung imprägniert ist, **dadurch gekennzeichnet, dass** die Zusammensetzung:
a) 0,05 Gew.-% bis 10 Gew.-% eines natürlichen Gelierungsmittels;
b) 1,0 Gew.-% bis 10 Gew.-% eines Hydrokolloids;
c) 5,0 Gew.-% bis 30,0 Gew.-% eines Alkylenglycols; und
d) mindestens 50 Gew.-% Wasser
umfasst.

2. Wundverband gemäß Anspruch 1, **dadurch gekennzeichnet, dass** die Zusammensetzung in der Form eines Gels vorliegt.

3. Wundverband gemäß Anspruch 1 oder 2, **dadurch gekennzeichnet, dass** das natürliche Gelierungsmittel Pektin ist.

4. Wundverband gemäß einem der vorstehenden Ansprüche, **dadurch gekennzeichnet, dass** das Hydrokolloid Natriumcarboxymethylcellulose ist.

5. Wundverband gemäß einem der vorstehenden Ansprüche, **dadurch gekennzeichnet, dass** das Alkylenglycol Propylenglycol ist.

6. Wundverbandmittel gemäß einem der vorstehenden Ansprüche, **dadurch gekennzeichnet, dass** das Gel eine Viskosität von 50 bis 800 Pas aufweist.

7. Verwendung eines faserförmigen Materials, das mit einer Zusammensetzung, umfassend:
a) 0,05 Gew.-% bis 10 Gew.-% eines natürlichen Gelierungsmittels;
b) 1,0 Gew.-% bis 10 Gew.-% eines Hydrokolloids;
c) 5,0 Gew.-% bis 30,0 Gew.-% eines Alkylenglycols; und
d) mindestens 50 Gew.-% Wasser
imprägniert ist, zur Herstellung eines Medikaments zur Verwendung bei der Behandlung von Wunden und insbesondere chronischen Wunden.

8. Verwendung gemäß Anspruch 7, **dadurch gekennzeichnet, dass** die Zusammensetzung in der Form eines Gels vorliegt.

9. Wundverbandprodukt, umfassend einen versiegelten Folienumschlag, enthaltend einen Wundverband, umfassend ein faserförmiges Material, das mit einer Zusammensetzung imprägniert ist, wobei die Zusammensetzung:
a) 0,05 Gew.-% bis 10 Gew.-% eines natürlichen Gelierungsmittels;
b) 1,0 Gew.-% bis 10 Gew.-% eines Hydrokolloids;
c) 5,0 Gew.-% bis 30,0 Gew.-% eines Alkylenglycols; und
d) mindestens 50 Gew.-% Wasser
umfasst.

10. Produkt gemäß Anspruch 9, **dadurch gekennzeichnet, dass** die Zusammensetzung in der Form eines Gels vorliegt.

11. Verfahren zur Herstellung eines Wundverbandes, umfassend die Schritte:
(i) Imprägnieren eines faserförmigen Materials mit einem Gel, wobei das Gel:
(a) 0,05 Gew.-% bis 10 Gew.-% eines natürlichen Gelierungsmittels;
(b) 1,0 Gew.-% bis 10 Gew.-% eines Hydrokolloids;
(c) 5,0 Gew.-% bis 30,0 Gew.-% eines Alkylenglycols; und
(d) mindestens 50 Gew.-% Wasser
umfasst;
(ii) Versiegeln des imprägnierten faserförmigen Materials in einen Umschlag und
(iii) Sterilisieren des Umschlags und des Inhalts.

12. Verfahren gemäß Anspruch 11, **dadurch gekennzeichnet, dass** die Sterilisation in einem Autoklaven durchgeführt wird.

13. Verfahren gemäß Anspruch 11, **dadurch gekennzeichnet, dass** die Sterilisation mit Gammabestrahlung durchgeführt wird.
